# EUROPÄISCHE PATENTSCHRIFT

(11) **EP 1 453 415 B1**
(45) Veröffentlichungstag und Bekanntmachung des Hinweises auf die Patenterteilung: **01.02.2006**
(21) Anmeldenummer: 03811373.4
(22) Anmeldetag: 12.11.2003
(51) Int. Cl.: A61B 1/313, A61B 1/00

(54) **ENDOSKOP**
ENDOSCOPE
ENDOSCOPE

(30) Priorität: 20.11.2002 DE 10255082
(43) Veröffentlichungstag der Anmeldung: 08.09.2004
(73) Patentinhaber: Aesculap AG & Co. KG, 78532 Tuttlingen (DE)
(72) Erfinder: ROTHWEILER, Chritstoph, 78166 Donaueschingen (DE); GROSSMANN, Andreas, 78532 Tuttligen (DE)
(74) Vertreter: Hoeger, Stellrecht & Partner Patentanwälte
(86) Internationale Anmeldenummer: PCT/EP2003/012634
(87) Internationale Veröffentlichungsnummer: WO 2004/045398

(56) Entgegenhaltungen:
- DE-A- 3 920 706
- US-A- 4 765 314
- US-A- 6 129 683

## Beschreibung

Die Erfindung betrifft ein Endoskop mit einem proximalen und einem in einen menschlichen Körper einführbaren distalen Endbereich, mit einem sich in einer Längsrichtung erstreckenden Schaft und mit einer den Schaft in Längsrichtung durchsetzenden, relativ zum Schaft bewegbaren Optik.

Endoskope der eingangs beschriebenen Art werden zu verschiedenen Zwecken im Rahmen minimal invasiver Eingriffe in den menschlichen Körper verwendet. Mit Hilfe der den Schaft durchsetzenden Optik kann ein Operateur den Operationsbereich einsehen. Bei herkömmlichen Endoskopen ist die Optik von proximal in den Schaft einführbar und in axialer Richtung verschiebbar. Als nachteilig hat sich hierbei erwiesen, daß die Optik unkontrolliert bewegt werden kann.

Aus der US 6,129,683 ist eine optische Biopsiezange mit einem Schaft und einer den Schaft in Längsrichtung durchsetzenden, relativ zum Schaft bewegbaren Optik bekannt. Femer wird in der US 4,765,314 eine Vorrichtung zum Einführen eines Endoskops oder eines chirurgischen Instruments in einen Körperhohlraum offenbart.

Es ist daher Aufgabe der vorliegenden Erfindung, ein Endoskop der eingangs genannten Art so zu verbessern, daß die Optik einfacher und sicherer handhabbar ist.

Diese Aufgabe wird bei einem gattungsgemäßen Endoskop dadurch gelöst, daß die Optik relativ zum Schaft in einer ersten Stellung und in mindestens einer zweiten, von der ersten Stellung verschiedenen Stellung in axialer Richtung festlegbar ist.

Diese Ausgestaltung ermöglicht es, daß die Optik zumindest in Längsrichtung des Schafts in genau definierten Positionen gehalten wird. Eine unerwünschte Relativbewegung zwischen dem Schaft und der Optik wird dadurch vermieden. Durch Auswahl von zwei Stellungen, beispielsweise einer Diagnosestellung und einer Arbeitsstellung, kann die Optik in gewünschter Weise eingesetzt werden. So kann sie in der Diagnosestellung zum Beispiel bis weit über das distale Ende des Endoskops hinausragen, ohne daß das distale Ende des Endoskops oder daran angeordnete Werkzeuge eine Sicht auf den Operationsbereich beeinträchtigen. Durch Überführung der Optik von der Diagnosestellung in die zweite, beispielsweise als Arbeitsstellung dienende Stellung, wird in definierter Weise eine optische Überwachung eines Einsatzes von am Schaft angeordneten Werkzeugen möglich. In der zweiten Stellung könnte ein freies Ende der Optik jedoch auch bis in den Schaft hinein zurückgezogen sein, so daß die Optik geschützt ist. Durch die Möglichkeit der axialen Festlegung der Optik relativ zum Schaft in zwei Stellungen läßt sich das Endoskop wesentlich einfacher und sicherer handhaben.

Besonders einfach läßt sich die Optik in axialer Richtung festlegen, wenn mindestens eine Rastvorrichtung zur Festlegung der Optik in der ersten und/oder der zweiten Stellung vorgesehen ist. Insbesondere kann die Optik dadurch automatisch bei Erreichen einer der beiden vorbestimmten Stellungen festgelegt werden. Durch entsprechende Ausgestaltung der Rastvorrichtung kann die festgelegte Optik auch wieder freigegeben und in axialer Richtung bewegt werden.

Vorzugsweise ist die Optik in der ersten Stellung und/oder in der zweiten Stellung relativ zum Schaft in Umfangsrichtung festgelegt. Dadurch ergibt sich eine vollständig definierte Stellung der Optik relativ zum Schaft.

Günstig ist es, wenn die Optik in einer dritten Stellung in axialer Richtung festlegbar und um die Längsachse des Schaftes frei rotierbar ist. In der dritten Stellung kann die Optik beispielsweise vollständig in das Innere des Schafts zurückgezogen sein, wodurch sie gegen äußere Einflüsse geschützt ist. Eine um die Längsachse des Schaftes freie Rotierbarkeit ermöglicht es, von einem proximalen Ende der Optik abgehende Anschlußleitungen beliebig zu orientieren.

Bei einer bevorzugten Ausführungsform der Erfindung kann vorgesehen sein, daß das distale Ende der Optik in der ersten Stellung über das distale Ende des Endoskops hervorsteht. Dadurch wird ein von der Optik einsehbarer Operationsbereich durch das Endoskop oder daran angeordnete Elemente nicht eingeschränkt.

Günstig ist es, wenn in der zweiten Stellung das distale Ende der Optik über das distale Ende des Schafts und das distale Ende des Endoskops über die Optik hervorsteht. Wenn die zweite Stellung derart vorgesehen ist, kann mittels der Optik das distale, beispielsweise durch vom Schaft abstehende Werkzeugelemente gebildete Ende des Endoskops eingesehen werden. Eine Bewegung von zur Anwendung kommenden Werkzeugelementen ist so optisch kontrollierbar.

Um herkömmliche Optiken in Verbindung mit einem erfindungsgemäßen Endoskop verwenden zu können, kann vorgesehen sein, daß das Endoskop eine Optikaufnahmevorrichtung umfaßt, daß die Optikaufnahmevorrichtung relativ zum Schaft bewegbar und in der ersten und/oder in der zweiten und/oder in der dritten Stellung in axialer Richtung festlegbar ist. Die mindestens eine Stellung, in der die Optik relativ zum Schaft in axialer Richtung festlegbar ist, wird nunmehr definiert durch eine entsprechende Ausgestaltung des Endoskops und der Optikaufnahmevorrichtung.

Vorzugsweise ist die Optik relativ zur Optikaufnahmevorrichtung um die Längsachse frei verdrehbar. Auf diese Weise kann die Optik auch bei axial festgelegter Optikaufnahmevorrichtung frei verdreht und ein Operationsbereich frei eingesehen werden. Ferner lassen sich von einem proximalen Ende der Optik abgehende Anschlußleitungen in gewünschter Weise orientieren.

Ein besonders einfacher Aufbau der Rastvorrichtung ergibt sich, wenn die Rastvorrichtung ein relativ zu einer Rastausnehmung bewegbares Rastglied umfaßt, wenn die Rastausnehmung oder das Rastglied an der Optik oder der Optikaufnahmevorrichtung angeordnet sind und wenn das Rastglied oder die Rastausnehmung an einer Aufnahme des Endoskops für die Optik oder die Optikaufnahmevorrichtung angeordnet sind. Eine Festlegung der jeweiligen Stellung wird erreicht, durch Eintauchen des Rastglieds in die Rastausnehmung. Umgekehrt ist ein Lösen auf einfache Weise dadurch erreichbar, daß das Rastglied aus der Rastausnehmung herausgeführt wird.

Gemäß einer weiteren bevorzugten Ausführungsform der Erfindung kann vorgesehen sein, daß das Endoskop einen Zuflußkanal zum Zuführen eines Fluids vom proximalen zum distalen Endbereich, einen Abflußkanal zum Abführen eines Fluids vom distalen zum proximalen Endbereich, einen mit dem Zuflußkanal in Strömungsverbindung stehenden Zuflußanschluß und einen mit dem Abfluß in Strömungsverbindung stehenden Abflußanschluß umfaßt. Aufgrund dieser Ausgestaltung kann ein Fluid über den Zuflußanschluß vom proximalen Ende zum distalen Ende des Endoskops geleitet werden und beispielsweise dort austreten, um den Operationsbereich zu spülen. Gleichzeitig kann durch den Abflußkanal das Fluid oder entferntes Körpergewebe abgeleitet werden.

Günstig ist es, wenn der Zuflußanschluß, der Abflußanschluß und die Optik relativ zueinander um eine im wesentlichen in Längsrichtung des Schafts verlaufende Drehachse drehbar sind. Damit lassen sich von den jeweiligen Anschlüssen abgehende Anschlußleitungen in einer beliebigen Stellung anordnen, wodurch auch die Gefahr vermindert wird, daß die Anschlußleitungen verdrillt oder miteinander verknotet werden.

Weiterhin ist es vorteilhaft, wenn der Zuflußanschluß, der Abflußanschluß und die Optik relativ zum Schaft um die im wesentlichen in Längsrichtung des Schafts verlaufende Drehachse drehbar sind. Der Schaft kann aufgrund dieser Ausgestaltung in das Körperinnere eingeführt werden und dort unverdrehbar liegenbleiben. Dagegen können vom Zuflußanschluß, vom Abflußanschluß oder von der Optik abgehende Leitungen beliebig orientiert werden, um einen Operateur nicht zu behindern oder ein Verdrillen der Leitungen zu verhindern. Durch den auf diese Weise innerhalb des Körpers ruhenden Schaft wird ferner eine Verletzungsgefahr für das Körperinnere aufgrund einer Rotation des Schafts praktisch ausgeschlossen.

Um einen Operationsbereich mit dem Endoskop nicht nur beobachten zu können, sondern zum eigentlichen Durchführen eines chirurgischen Eingriffs, ist es günstig, wenn am distalen Ende des Schafts mindestens ein bewegliches Werkzeug angeordnet ist. Das Werkzeug kann beispielsweise eine Fasszange, eine Schere oder dergleichen sein.

Vorteilhaft ist es, wenn das bewegliche Werkzeug relativ zum Schaft um eine im wesentlichen in Längsrichtung des Schaftes verlaufende Drehachse verdrehbar ist. Damit kann eine relative Stellung des beweglichen Werkzeugs relativ zu einem im Körperinneren feststehenden Schaft beliebig verändert werden.

Bei einer bevorzugten Ausführungsform der Erfindung kann vorgesehen sein, daß das Werkzeug mindestens ein relativ zum Schaft bewegbar gelagertes Maulteil umfaßt. So läßt sich auf einfache Weise eine Schere oder eine Fasszange ausbilden.

Um einen Aktionsbereich des Werkzeugs zu vergrößern, ist es günstig, wenn mindestens zwei zueinander bewegbare Maulteile vorgesehen sind. Diese können beispielsweise bis weit über einen Querschnittsbereich des Schafts nach außen verschwenkt werden und Körpergewebe erfassen, insbesondere bei der Entfernung von Septen, Polypen oder dergleichen, die teilweise im Querschnitt größer sind als der Querschnitt des ins Körperinnere eingeführten Schafts.

Vorteilhaft ist es, wenn das mindestens eine bewegbare Maulteil relativ zum Schaft um eine im wesentlichen quer zur Längsrichtung des Schafts verlaufende Schwenkachse verschwenkbar ist. Dadurch läßt sich eine Schwenkbewegung des Maulteils auf besonders einfache Weise realisieren, insbesondere eine seitliche Verschwenkung des Maulteils.

Vorzugsweise ist eine dem mindestens einen Maulteil zugeordnete Betätigungsvorrichtung vorgesehen und daß die Betätigungsvorrichtung relativ zum Schaft verdrehbar ist. Mit der Betätigungsvorrichtung läßt sich eine Bewegung des Maulteils realisieren. Aufgrund der Verdrehbarkeit der Betätigungsvorrichtung relativ zum Schaft kann von der Stellung der Betätigungsvorrichtung auf die relative Orientierung des zugehörigen Maulteils geschlossen werden.

Um beispielsweise eine Fasszange in einer geschlossenen Stellung zu halten, ist es günstig, wenn die Betätigungsvorrichtung in einer Betätigungsstellung des mindestens einen beweglichen Maulteils verriegelbar ist. Eine Verriegelung könnte jedoch auch in einer geöffneten Stellung der Maulteile denkbar sein.

Günstig ist es, wenn mindestens zwei Maulteile vorgesehen sind, welche in einer geschlossenen Stellung, in der die beiden Maulteile aneinander anliegen, symmetrisch zu einer die Längsachse des Schafts enthaltenden Ebene ausgebildet sind. Unabhängig davon, ob nur eines der beiden Maulteile oder beide beweglich am Schaft gelagert sind, ergibt sich dadurch ein besonders einfacher Aufbau des Endoskops.

Vorzugsweise ist die Optik im Bereich der mindestens zwei Maulteile auf einer Seite des feststehenden Maulteils angeordnet und das bewegliche Maulteil auf der der Optik abgewandten Seite des feststehenden Maulteils. Die Optik ist so außerhalb des Arbeitsbereichs der beiden Maulteile angeordnet und kann nicht zwischen diese geraten und dadurch beschädigt werden. Sie ist vielmehr auf diese Weise geschützt angeordnet.

Ein besonders guter Schutz der Optik ergibt sich, wenn das feststehende Maulteil in Richtung der Optik weisend konkav gekrümmt ist. Die Optik kann sich so an den konkaven Bereich des Maulteils anschmiegen. Dadurch wird auch der Querschnitt des ins Körperinnere einführbaren Abschnitts des Endoskops minimiert.

Gemäß einer weiteren bevorzugten Ausführungsform der Erfindung kann vorgesehen sein, daß der Schaft einen dem distalen Ende des Endoskops benachbarten, in einen menschlichen Körper einführbaren Schaftabschnitt umfaßt, daß die Symmetrieachse der Optik im Bereich des Schaftabschnittes parallel zur Symmetrieachse des Schaftes im Bereich des Schaftabschnittes verläuft und daß beide Symmetrieachsen voneinander beabstandet sind. Eine derartige exzentrische Anordnung der Längsachsen des Schaftabschnittes und der Optik ermöglicht es, daß der Schaft und insbesondere an diesem angeordnete Werkzeuge um die Optik herum verdrehbar sind. Damit bildet die Längsachse der Optik insgesamt auch die Drehachse für den gesamten, in das Körperinnere einführbaren Bereich des Endoskops.

Für einen Einsatz des Endoskops im Bereich der Hochfrequenz-Chirurgie ist es von Vorteil, wenn eine HF-Anschlußvorrichtung zum Verbinden des Endoskops mit einem Hochfrequenzgenerator vorgesehen ist und wenn diese Anschlußvorrichtung relativ zur Optik drehbar angeordnet ist. Dadurch lassen sich ferner auch Anschlußleitungen in gewünschter Orientierung anordnen und ein Verdrillen der Anschlußleitungen wird verhindert.

Die nachfolgende Beschreibung einer bevorzugten Ausführungsform der Erfindung dient im Zusammenhang mit der Zeichnung der näheren Erläuterung. Es zeigen :
- Figur 1:: eine teilweise geschnittene Seitenansicht eines Endoskops;
- Figur 2:: eine vergrößerte Schnittansicht des Bereichs A in Figur 1;
- Figur 3:: eine Querschnittansicht längs Linie 3-3 in Figur 2;
- Figur 4:: eine teilweise durchbrochene Draufsicht auf ein Endoskop mit einer Optik in einer Diagnosestellung;
- Figur 5:: eine Seitenansicht des in Figur 4 dargestellten Endbereichs des Endoskops;
- Figur 6:: eine Draufsicht ähnlich Figur 4 mit der Optik in einer Arbeitsstellung; und
- Figur 7:: eine Draufsicht ähnlich Figur 4 mit der Optik in einer zurückgezogenen Stellung.

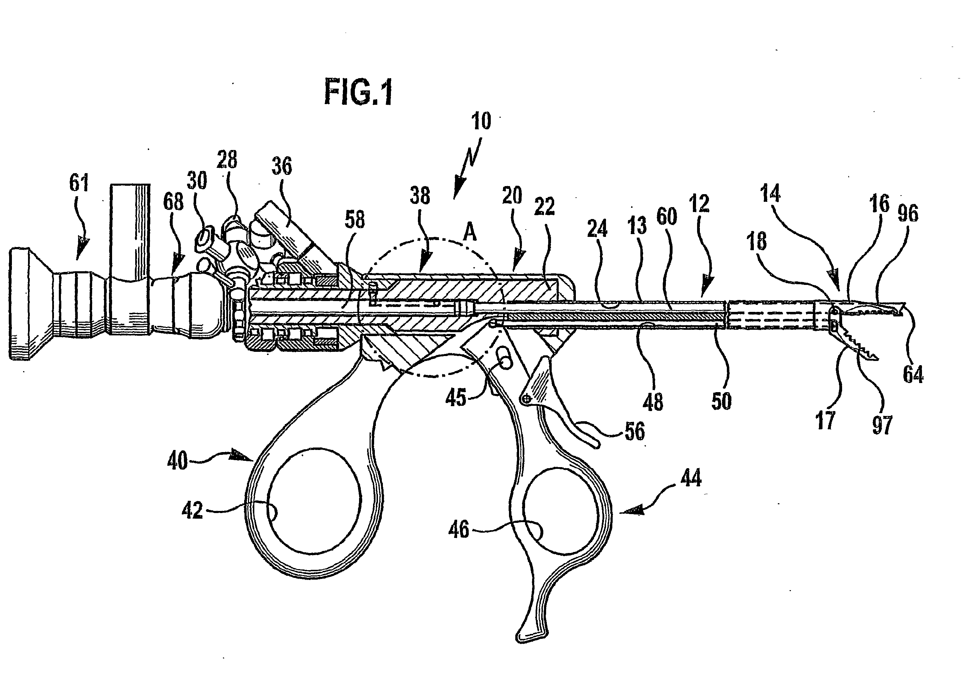

In Figur 1 ist ein insgesamt mit dem Bezugszeichen 10 versehenes Endoskop dargestellt, mit einem durch eine Körperöffnung ins Innere eines menschlichen Körpers einführbaren Schaft 12, an dessen distalem Ende ein chirurgisches Werkzeug in Form einer Fasszange 14 relativ zum Schaft um dessen Längsachse rotierbar angeordnet ist. Die Fasszange umfaßt ein feststehendes Maulteil 16 sowie ein um eine quer zur Längsachse des Schafts 12 verlaufende Schwenkachse 18 verschwenkbar gelagertes Maulteil 17.

An ein proximales Ende des Schafts 12 schließt sich ein Handhabungsbereich 20 des Endoskops 10 an, welcher einen mit einer äußeren Schafthülse 13 des Schafts 12 starr verbundenen Grundkörper 22 umfaßt. Durch den Schaft 12 und den Grundkörper 22 erstreckt sich ein Optikkanal 24, der sich im Bereich des Grundkörpers 22 in Richtung auf dessen proximales Ende hin einstufig erweitert, so daß ein ringförmiger Anschlag 26 ausgebildet ist. Im proximalen Endbereich des Grundkörpers 22 sind um diesen und relativ zueinander rotierbar ein Zuflußanschluß 28 und ein Abflußanschluß 30 vorgesehen, die jeweils mit einem im Querschnitt linsenförmigen, sich durch den Grundkörper 22 und den Schaft 12 erstreckenden Zuflußkanal 32 bzw. Abflußkanal 34 in Fluidverbindung stehen. Ebenfalls drehbar am Grundkörper 22 gelagert ist ein HF-Anschluß 36, der über nicht dargestellte elektrische Verbindungen, welche sich in Längsrichtung durch den Schaft 12 erstrecken, mit den beiden Maulteilen 16 und 17 leitend in Verbindung steht.

Um einen distalen Bereich des Grundkörpers 22 rotierbar ist ein Griffbereich 38 angeordnet, welcher eine feststehende, seitlich vom Grundkörper 22 in proximaler Richtung weisend abstehende Branche 40 mit einer Daumenöffnung 20 und eine am Griffbereich 38 relativ zur Branche 40 um eine quer zur Längsachse des Schafts 12 verlaufende Schwenkachse 45 verschwenkbare Branche 44 mit einer Fingeröffnung 46 umfaßt. In einen sich in Längsrichtung des Schafts 12 erstreckenden und diesen durchsetzenden Manipulationskanal 48 ist eine Schub- und Zugstange 50 eingesetzt, deren proximales Ende beweglich an der Branche 44 gelagert und deren distales Ende über seitlich abstehende, in Führungsschlitze 52 des Maulteils 17 eintauchende Lagerzapfen 54 beweglich mit dem Maulteil 17 verbunden ist. Durch eine Verschwenkbewegung der Branche 44 wird die Schub- und Zugstange 50 in Längsrichtung des Schafts 12 bewegt, wodurch das Maulteil 17 relativ zum Maulteil 16 verschwenkt wird.

Mit dem Verriegelungshebel 56 läßt sich eine Schwenkstellung der Branche 44 relativ zur Branche 40 arretieren, wodurch das bewegliche Maulteil 17 in einer gewünschten Stellung gehalten wird, beispielsweise in Anlage an das feststehende Maulteil 16 oder in einer offenen Stellung, bei der es seitlich vom Maulteil 16 abgespreizt ist.

In den Optikkanal 24 ist proximalseitig eine Führungshülse 58 eingesetzt, die mit ihrem distalen Ende nahezu am Anschlag 26 anliegt. Die Führungshülse 58 ist Teil eines Optikadapters 68 über den ein proximalseitiger Anschlußbereich 61 einer Optik 60 mit weiteren optischen Geräten verbindbar ist, beispielsweise einer Kamera oder einem Mikroskop oder dergleichen. Durch eine Verbindung zwischen dem Optikadapter 68 und dem Anschlußbereich 61 läßt sich eine axiale Festlegung der beiden Elemente, beispielsweise über eine lösbare Rastverbindung erreichen. Eine relative Axialbewegung zwischen der Führungshülse 58 und der Optik 60 wird dadurch im verbundenen Zustand verhindert. Falls gewünscht, kann die Rastverbindung so ausgelegt werden, daß eine Rotation des Anschlußbereichs 61 relativ zum Optikadapter 68 möglich ist.

Durch die Führungshülse 58 hindurch kann vom proximalen Ende des Endoskops 10 her die stabförmige Optik 60 eingeschoben werden, welche ein relativ zu ihrer Längsachse 62 abgeschrägtes Ende 64 aufweist, welches durch eine relativ zur Längsachse 62 um einen Neigungswinkel 66 geneigte Fläche gebildet wird.

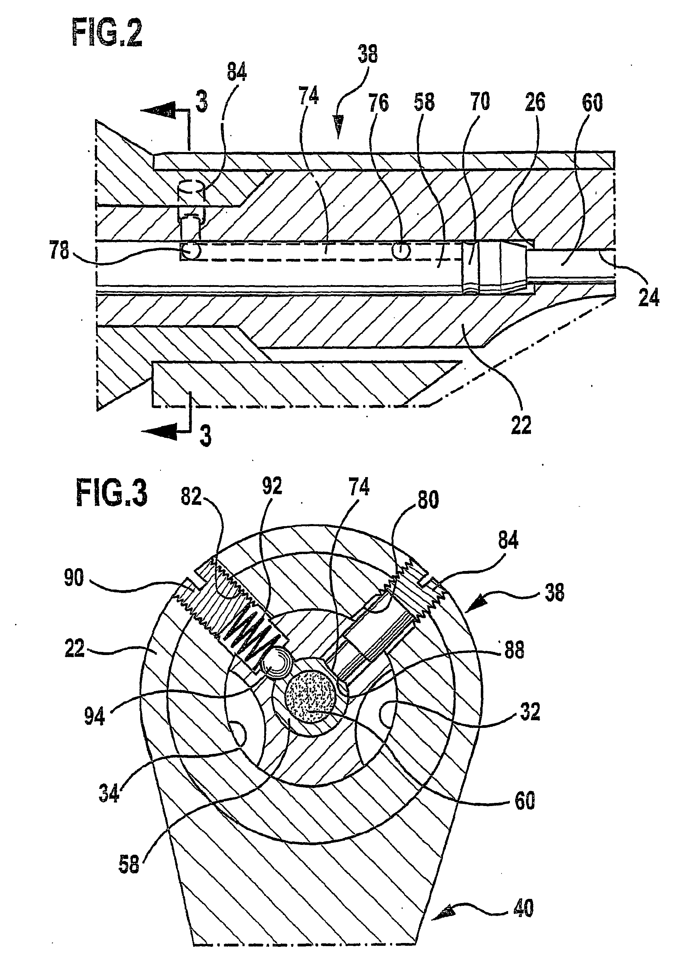

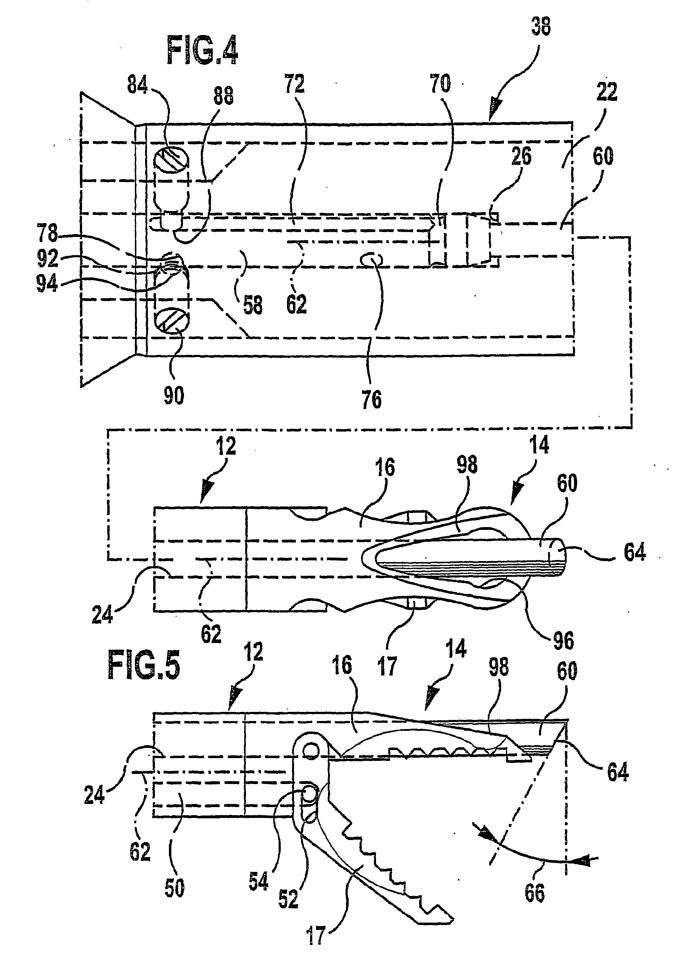

Die Führungshülse 58 ist, wie in den Figuren 2, 3 und 6 dargestellt, benachbart ihrem distalen Ende mit einer Ringnut 70 versehen, von der ausgehend sich in proximaler Richtung entlang ihrer äußeren Oberfläche eine Abflachung 74 in etwa über die Hälfte der Länge der Führungshülse 58 erstreckt. Alternativ kann, wie in Figur 4 dargestellt, anstelle der Abflachung 74 auch eine Längsnut 72 vorgesehen sein. Um 90° in Umfangsrichtung versetzt sind an der Führungshülse 58 voneinander in Längsrichtung beabstandet zwei hohlkugelige Vertiefungen 76 und 78 eingelassen, wobei der Abstand zwischen der Vertiefung 76 und der Vertiefung 78 in etwa der Länge des beweglichen Maulteils 17 entspricht. Der Abstand zwischen der Vertiefung 76 und der Ringnut 70 ist dagegen deutlich kleiner.

Zur Führung der Führungshülse 58 ist der Griffbereich 38 mit zwei radialen, in Umfangsrichtung um etwa 90° versetzt zueinander angeordneten Gewindebohrungen 80 und 82 versehen. In die Gewindebohrung 80 ist ein Gewindestift 84 eingesetzt, dessen Ende 88 entweder, wie beispielsweise in Figur 4 dargestellt, korrespondierend zur Längsnut 72 ausgebildet ist oder der ein abgeflachtes Ende 88 korrespondierend zur Abflachung 74 aufweist, wie insbesondere in Figur 3 dargestellt. In die Gewindebohrung 82 ist eine Madenschraube 90 eingesetzt, an der sich eine Spiralfeder 92 in Richtung auf die Führungshülse 58 hin abstützt und eine Kugel 94 gegen die Führungshülse 58 drückt. In Kombination bilden die Madenschraube 90, die Spiralfeder 92 und die Kugel 94 ein Kugeldruckstück, über das eine Rastverbindung mit den beiden, an der Führungshülse 58 vorgesehenen, Rastausnehmungen bildenden Vertiefungen 76 und 78 hergestellt werden kann. Der Gewindestift 84 wird in die Gewindebohrung 80 so weit eingeschraubt, daß das Ende 88 in die Längsnut 72 eintaucht und dadurch die Führungshülse 58 in Längsrichtung derselben geführt wird. Die Führungshülse 58 wird in axialer Richtung relativ zum Griffbereich 38 und damit zum Schaft 12 festgelegt, wenn die Kugel 94 in eine der beiden Vertiefungen 76 oder 78 oder in die Ringnut 70 eintaucht. Im letztgenannten Fall ist eine Rotation des Griffbereichs 38 um den Optikadapter 68 möglich.

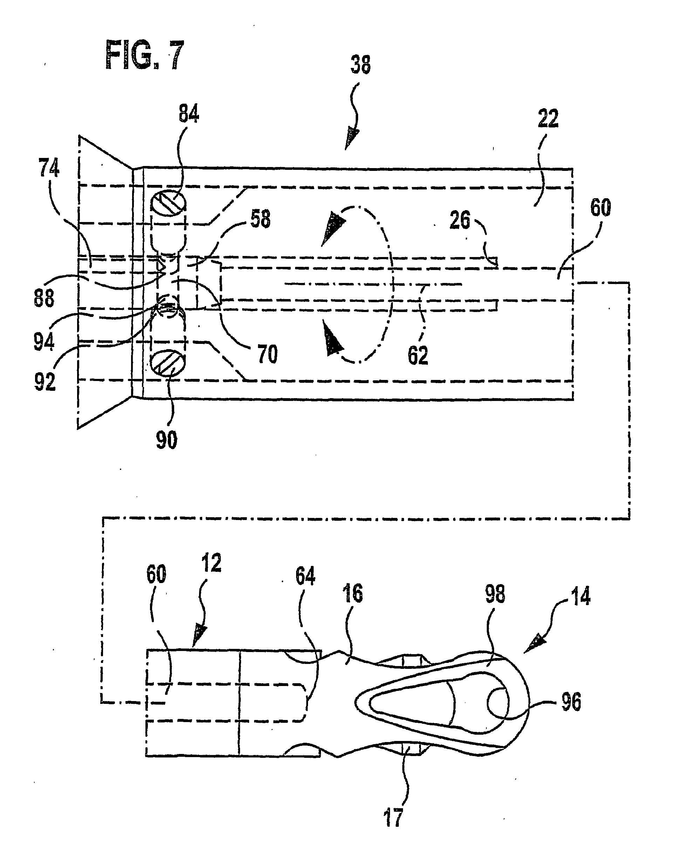

Aufgrund der Ausbildungen der beiden Vertiefungen 76 und 78 sowie der Ringnut 70 ist der Optikadapter 68 relativ zum Schaft 12 in drei axialen Stellungen festlegbar, die im Zusammenhang mit den Figuren 4 bis 7 näher erläutert werden.

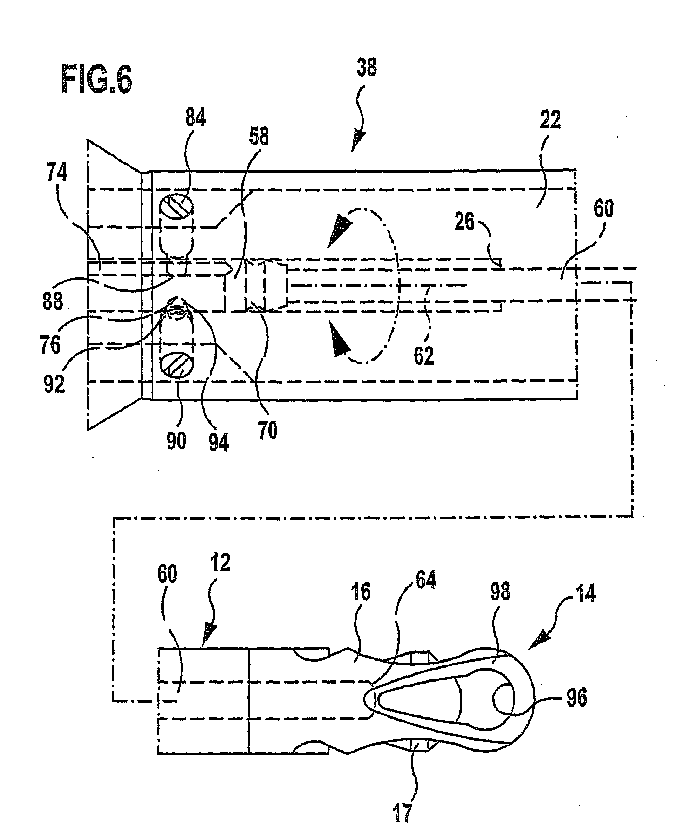

In der sogenannten Diagnosestellung taucht die Kugel 94 in die Vertiefung 78 ein. Das Ende 64 der Optik 60 ragt in der Diagnosestellung etwas über das Ende des feststehenden Maulteils 16 in distaler Richtung hinaus. Auf diese Weise kann ein Operationsbereich eingesehen werden, ohne daß die beiden Maulteile 16 und 17 die Sicht beeinträchtigen. Dies ist in den Figuren 4 und 5 dargestellt. In der Arbeitsstellung, die in Figur 6 dargestellt ist, taucht die Kugel 94 in die Vertiefung 76 ein. Das Ende 64 der Optik 60 ragt in dieser Stellung etwas über den Optikkanal 24 aus dem Schaft 12 hervor, jedoch nicht über das distale Ende des feststehenden Maulteils 16 hinaus. In dieser Arbeitsstellung läßt sich mit der Optik 60 ein mittels der Maulteile 16 und 17 vorgenommener chirurgischer Eingriff beobachten.

Wird der Optikadapter 68 in axialer Richtung noch weiter zurückgezogen, taucht die Kugel 94 schließlich in die Ringnut 70 ein, in die gleichzeitig auch das freie Ende 88 des Gewindestifts 84 eintaucht, wodurch eine Rotation der Führungshülse 58 relativ zum Griffbereich 38 ermöglicht wird. In dieser dritten Stellung des Optikadapters 68 ist das Ende 64 der Optik 60 in den Optikkanal 24 hinein zurückgezogen, so daß das Ende 64 geschützt ist.

Die Längsachse 62 der Optik 60 ist im Bereich des Schafts 12 zu dessen Längsachse parallel versetzt, wodurch sich insgesamt eine exzentrische Rotation des Schafts 12 um die Optik 60 ergibt. Die Optik 60 schmiegt sich im Bereich des feststehenden Maulteils 16, welches ebenso wie das Maulteil 17 mit einer langgestreckten Querdurchbrechung 96 bzw. 97 versehen ist, in eine radial nach außen wegweisende konkave Vertiefung 98 des feststehenden Maulteils 16 an. Damit wird die Optik 60 auf einer dem beweglichen Maulteil 17 abgewandten Seite des feststehenden Maulteils geführt. Eine Beschädigung der Optik 60 durch die beiden Maulteile 16 und 17 wird dadurch vermieden.

## Patentansprüche

1. Endoskop mit einem proximalen und einem in einen menschlichen Körper einführbaren distalen Endbereich, mit einem sich in einer Längsrichtung erstreckenden Schaft und mit einer den Schaft in Längsrichtung durchsetzenden, relativ zum Schaft bewegbaren Optik, **dadurch gekennzeichnet, daß** die Optik (60) relativ zum Schaft (12) in einer ersten Stellung und in mindestens einer zweiten, von der ersten Stellung verschiedenen Stellung in axialer Richtung festlegbar ist.

2. Endoskop nach Anspruch 1, **dadurch gekennzeichnet, daß** mindestens eine Rastvorrichtung (70, 76, 78, 90, 92, 94) zur Festlegung der Optik (60) in der ersten und/oder der zweiten Stellung vorgesehen ist.

3. Endoskop nach einem der voranstehenden Ansprüche, **dadurch gekennzeichnet, daß** die Optik (60) in der ersten Stellung und/oder der zweiten Stellung relativ zum Schaft (12) in Umfangsrichtung festgelegt ist.

4. Endoskop nach einem der voranstehenden Ansprüche, **dadurch gekennzeichnet, daß** die Optik (60) in einer dritten Stellung in axialer Richtung festlegbar und um die Längsachse des Schaftes (12) frei rotierbar ist.

5. Endoskop nach einem der voranstehenden Ansprüche, **dadurch gekennzeichnet, daß** das distale Ende (64) der Optik (60) in der ersten Stellung über das distale Ende (16) des Endoskops (10) hervorsteht.

6. Endoskop nach einem der voranstehenden Ansprüche, **dadurch gekennzeichnet, daß** in der zweiten Stellung das distale Ende (64) der Optik (60) über das distale Ende Schafts (12) und das distale Ende des Endoskops (10) über die Optik (60) hervorsteht.

7. Endoskop nach einem der voranstehenden Ansprüche, **dadurch gekennzeichnet, daß** das Endoskop (10) eine Optikaufnahmevorrichtung (68) umfasst, daß die Optikaufnahmevorrichtung (68) relativ zum Schaft (12) bewegbar und in der ersten und/oder in der zweiten und/oder in der dritten Stellung in axialer Richtung festlegbar ist.

8. Endoskop nach Anspruch 7, **dadurch gekennzeichnet, daß** die Optik (60) relativ zur Optikaufnahmevorrichtung (68) um die Längsachse (62) der Optik (60) frei verdrehbar ist.

9. Endoskop nach einem der Ansprüche 2 bis 8, **dadurch gekennzeichnet, daß** die Rastvorrichtung ein relativ zu einer Rastausnehmung (70, 76, 78) bewegbares Rastglied (94) umfasst, daß die Rastausnehmung (70, 76, 78) oder das Rastglied (94) an der Optik (60) oder der Optikaufnahmevorrichtung (68) angeordnet sind und daß das Rastglied (94) oder die Rastausnehmung (70, 76, 78) an einer Aufnahme (24) des Endoskops (10) für die Optik (60) oder die Optikaufnahmevorrichtung (68) angeordnet sind.

10. Endoskop nach einem der voranstehenden Ansprüche, **dadurch gekennzeichnet, daß** das Endoskop (10) einen Zuflußkanal (32) zum Zuführen eines Fluids vom proximalen zum distalen Endbereich, einen Abflußkanal (34) zum Abführen eines Fluids vom distalen zum proximalen Endbereich, einen mit dem Zuflußkanal (32) in Strömungsverbindung stehenden Zuflußanschluß (28) und einen mit dem Abflußkanal (34) in Strömungsverbindung stehenden Abflußanschluß (30) umfaßt.

11. Endoskop nach Anspruch 10, **dadurch gekennzeichnet, daß** der Zuflußanschluß (28), der Abflußanschluß (30) und die Optik (60) relativ zueinander um eine im Wesentlichen in Längsrichtung des Schafts (12) verlaufende Drehachse (62) drehbar sind.

12. Endoskop nach einem der Ansprüche 10 oder 11, **dadurch gekennzeichnet, daß** der Zuflußanschluß (28), der Abflußanschluß (30) und die Optik (60) relativ zum Schaft (12) um die im Wesentlichen in Längsrichtung des Schafts (12) verlaufende Drehachse (62) drehbar sind.

13. Endoskop nach einem der voranstehenden Ansprüche, **dadurch gekennzeichnet, daß** am distalen Ende des Schaftes (12) mindestens ein bewegliches Werkzeug (16, 17) angeordnet ist.

14. Endoskop nach Anspruch 13, **dadurch gekennzeichnet, daß** das beweg liche Werkzeug (16, 17) relativ zum Schaft (12) um eine im Wesentlichen in Längsrichtung des Schafts (12) verlaufende Drehachse verdrehbar ist.

15. Endoskop nach einem der Ansprüche 13 oder 14, **dadurch gekennzeichnet, daß** das Werkzeug mindestens ein relativ zum Schaft (12) bewegbar gelagertes Maulteil (17) umfaßt.

16. Endoskop nach Anspruch 15, **dadurch gekennzeichnet, daß** mindestens zwei relativ zueinander bewegbare Maulteile (16, 17) vorgesehen sind.

17. Endoskop nach einem der Ansprüche 15 oder 16, **dadurch gekennzeichnet, daß** das mindestens eine bewegbare Maulteil (17) relativ zum Schaft (12) um eine im Wesentlichen quer zur Längsrichtung des Schafts (12) verlaufende Schwenkachse (18) verschwenkbar ist.

18. Endoskop nach einem der Ansprüche 15 bis 17, **dadurch gekennzeichnet, daß** eine dem mindestens einen Maulteil (17) zugeordnete Betätigungsvorrichtung (44, 50) vorgesehen ist und daß die Betätigungsvorrichtung (44, 50) relativ zum Schaft (12) verdrehbar ist.

19. Endoskop nach Anspruch 18, **dadurch gekennzeichnet, daß** die Betätigungsvorrichtung (44, 50) in einer Betätigungsstellung des mindestens einen beweglichen Maulteils (17) verriegelbar ist.

20. Endoskop nach einem der Ansprüche 15 bis 19, **dadurch gekennzeichnet, daß** mindestens zwei Maulteile (16, 17) vorgesehen sind, welche in einer geschlossenen Stellung, in der die beiden Maulteile (16, 17) aneinander anliegen, symmetrisch zu einer die Längsachse des Schafts (12) enthaltenden Ebene ausgebildet sind.

21. Endoskop nach Anspruch 20, **dadurch gekennzeichnet, daß** die Optik (60) im Bereich der mindestens zwei Maulteile (16, 17) auf einer Seite eines feststehenden Maulteils (16) angeordnet ist und daß das bewegliche Maulteil (17) auf der der Optik (60) abgewandten Seite des feststehenden Maulteils (16) angeordnet ist.

22. Endoskop nach Anspruch 21, **dadurch gekennzeichnet, daß** das feststehende Maulteil (16) in Richtung der Optik (60) weisend konkav gekrümmt ist.

23. Endoskop nach einem der voranstehenden Ansprüche, **dadurch gekennzeichnet, daß** der Schaft einen dem distalen Ende des Endoskops (10) benachbarten, in einen menschlichen Körper einführbaren Schaftabschnitt (12) umfaßt, daß die Symmetrieachse (62) der Optik im Bereich des Schaftabschnittes (12) parallel zur Symmetrieachse des Schaftes (12) im Bereich des Schaftabschnittes (12) verläuft und daß beide Symmetrieachsen voneinander beabstandet sind.

24. Endoskop nach einem der voranstehenden Ansprüche, **dadurch gekennzeichnet, daß** eine HF-Anschlussvorrichtung (36) zum Verbinden des Endoskops (10) mit einem Hochfrequenzgenerator vorgesehen ist und daß die Anschlußvorrichtung (36) relativ zur Optik (60) drehbar angeordnet ist.

## Claims

1. Endoscope having a proximal end area, a distal end area introducible into the body of a human being, a shaft extending in a longitudinal direction, and an optical device extending through the shaft in the longitudinal direction thereof and movable relative to the shaft, **characterized in that** the optical device (60) is fixable in axial direction relative to the shaft (12) in a first position and in at least a second position differing from the first position.

2. Endoscope in accordance with claim 1, **characterized in that** at least one locking device (70, 76, 78, 90, 92, 94) is provided for fixing the optical device (60) in the first position and/or the second position.

3. Endoscope in accordance with any one of the preceding claims, **characterized in that** in the first position and/or the second position, the optical device (60) is fixed in circumferential direction relative to the shaft (12).

4. Endoscope in accordance with any one of the preceding claims, **characterized in that** in a third position, the optical device (60) is fixable in axial direction and freely rotatable about the longitudinal axis of the shaft (12).

5. Endoscope in accordance with any one of the preceding claims, **characterized in that** in the first position, the distal end (64) of the optical device (60) projects over the distal end (16) of the endoscope (10).

6. Endoscope in accordance with any one of the preceding claims, **characterized in that** in the second position, the distal end (64) of the optical device (60) projects over the distal end of the shaft (12), and the distal end of the endoscope (10) projects over the optical device (60).

7. Endoscope in accordance with any one of the preceding claims, **characterized in that** the endoscope (10) comprises a device for receiving the optical device (68), and **in that** the device for receiving the optical device (68) is movable relative to the shaft (12) and fixable in axial direction in the first position and/or in the second position and/or in the third position.

8. Endoscope in accordance with claim 7, **characterized in that** the optical device (60) is freely rotatable about the longitudinal axis (62) of the optical device (60) relative to the device for receiving the optical device (68).

9. Endoscope in accordance with any one of claims 2 to 8, **characterized in that** the locking device comprises a locking member (94) movable relative to a locking recess (70, 76, 78), **in that** the locking recess (70, 76, 78) or the locking member (94) is arranged on the optical device (60) or on the device for receiving the optical device (68), and **in that** the locking member (94) or the locking recess (70, 76, 78) is arranged on a receptacle (24) of the endoscope (10) for the optical device (60) or for the device for receiving the optical device (68).

10. Endoscope in accordance with any one of the preceding claims, **characterized in that** the endoscope (10) comprises an inflow channel (32) for feeding a fluid from the proximal end area to the distal end area, an outflow channel (34) for conducting a fluid away from the distal end area to the proximal end area, an inflow connection (28) in flow communication with the inflow channel (32), and an outflow connection (30) in flow communication with the outflow channel (34).

11. Endoscope in accordance with claim 10, **characterized in that** the inflow connection (28), the outflow connection (30) and the optical device (60) are rotatable relative to one another about an axis of rotation (62) extending substantially in the longitudinal direction of the shaft (12).

12. Endoscope in accordance with claim 10 or 11, **characterized in that** the inflow connection (28), the outflow connection (30) and the optical device (60) are rotatable relative to the shaft (12) about the axis of rotation (62) extending substantially in the longitudinal direction of the shaft (12).

13. Endoscope in accordance with any one of the preceding claims, **characterized in that** at least one movable tool (16, 17) is arranged at the distal end of the shaft (12).

14. Endoscope in accordance with claim 13, **characterized in that** the movable tool (16, 17) is rotatable relative to the shaft (12) about an axis of rotation extending substantially in the longitudinal direction of the shaft (12).

15. Endoscope in accordance with claim 13 or 14, **characterized in that** the tool comprises at least one jaw part (17) which is mounted for movement relative to the shaft (12).

16. Endoscope in accordance with claim 15, **characterized in that** at least two jaw parts (16, 17) movable relative to each other are provided.

17. Endoscope in accordance with claim 15 or 16, **characterized in that** the at least one movable jaw part (17) is pivotable relative to the shaft (12) about a pivot axis (18) extending substantially transversely to the longitudinal direction of the shaft (12).

18. Endoscope in accordance with any one of claims 15 to 17, **characterized in that** an actuating device (44, 50) associated with the at least one jaw part (17) is provided, and **in that** the actuating device (44, 50) is rotatable relative to the shaft (12).

19. Endoscope in accordance with claim 18, **characterized in that** the actuating device (44, 50) is lockable in an actuated position of the at least one movable jaw part (17).

20. Endoscope in accordance with any one of claims 15 to 19, **characterized in that** at least two jaw parts (16, 17) are provided, which, in a closed position in which the two jaw parts (16, 17) rest against each other, are formed symmetrically in relation to a plane containing the longitudinal axis of the shaft (12).

21. Endoscope in accordance with claim 20, **characterized in that** the optical device (60) is arranged in the area of the at least two jaw parts (16, 17) on a side of a stationary jaw part (16), and **in that** the movable jaw part (17) is arranged at that side of the stationary jaw part (16) which faces away from the optical device (60).

22. Endoscope in accordance with claim 21, **characterized in that** the stationary jaw part (16) is of concavely curved configuration in the direction pointing towards the optical device (60).

23. Endoscope in accordance with any one of the preceding claims, **characterized in that** the shaft has adjacent to the distal end of the endoscope (10) a shaft section (12) which is introducible into the body of a human being, **in that** the axis of symmetry (62) of the optical device extends in the area of the shaft section (12) parallel to the axis of symmetry of the shaft (12) in the area of the shaft section (12), and **in that** the two axes of symmetry are spaced from each other.

24. Endoscope in accordance with any one of the preceding claims, **characterized in that** an HF connecting device (36) is provided for connecting the endoscope (10) to a high-frequency generator, and **in that** the connecting device (36) is arranged for rotation relative to the optical device (60).

## Revendications

1. Endoscope comportant une extrémité proximale et une extrémité distale susceptible d'être introduite dans un corps humain, un tube s'étendant dans une direction longitudinale et une optique traversant le tube dans la direction longitudinale, mobile par rapport à ce dernier, **caractérisé en ce que** l'optique (60) peut être immobilisée par rapport au tube (12) dans une première position et au moins dans une deuxième position, différente de la première, dans la direction axiale.

2. Endoscope selon la revendication 1, **caractérisé en ce qu'**est prévu au moins un dispositif à encoches (70, 76, 78, 90, 92, 94) pour immobiliser l'optique (60) dans la première et/ou la deuxième position.

3. Endoscope selon l'une des revendications précédentes, **caractérisé en ce que** l'optique (60) est immobilisée par rapport au tube (12) dans la première et/ou la deuxième position dans la direction du périmètre.

4. Endoscope selon l'une des revendications précédentes, **caractérisé en ce que** l'optique (60) peut être immobilisée dans une troisième position dans la direction axiale et peut tourner librement autour de l'axe longitudinal du tube (12).

5. Endoscope selon l'une des revendications précédentes, **caractérisé en ce que**, dans la première position, l'extrémité distale (64) de l'optique (60) dépasse de l'extrémité distale (16) de l'endoscope (10).

6. Endoscope selon l'une des revendications précédentes, **caractérisé en ce que**, dans la deuxième position, l'extrémité distale (64) de l'optique (60) dépasse de l'extrémité distale du tube (12) et l'extrémité distale de l'endoscope (10) dépasse de l'optique (60).

7. Endoscope selon l'une des revendications précédentes, **caractérisé en ce que** l'endoscope (10) comprend un dispositif de logement de l'optique (68), que le dispositif de logement de l'optique (68) est mobile par rapport au tube (12) et peut être immobilisé dans la première et/ou la deuxième et/ou la troisième position dans la direction axiale.

8. Endoscope selon la revendication 7, **caractérisé en ce que** l'optique (60) peut tourner librement autour de l'axe longitudinal (62) de l'optique (60), par rapport au dispositif de logement de l'optique (68).

9. Endoscope selon l'une des revendications 2 à 8, **caractérisé en ce que** le dispositif à encoches comprend un élément d'arrêt (94) mobile par rapport à une encoche (70, 76, 78), que l'encoche (70, 76, 78) ou l'élément d'arrêt (94) sont disposés sur l'optique (60) ou sur le dispositif de logement de l'optique (68) et que l'élément d'arrêt (94) ou l'encoche (70, 76, 78) sont disposés sur un logement (24) de l'endoscope (10) destiné à l'optique (60) ou au dispositif de logement de l'optique (68).

10. Endoscope selon l'une des revendications précédentes, **caractérisé en ce que** l'endoscope (10) comprend un conduit d'admission (32) destiné à amener un fluide de l'extrémité proximale à l'extrémité distale, un conduit de décharge (34) destiné à évacuer un fluide de l'extrémité distale à l'extrémité proximale, un raccord d'admission (28) en liaison d'écoulement avec le conduit d'admission (32) et un raccord de décharge (30) en liaison d'écoulement avec le conduit de décharge (34).

11. Endoscope selon la revendication 10, **caractérisé en ce que** le raccord d'admission (28), le raccord de décharge (30) et l'optique (60) peuvent tourner les uns par rapport aux autres autour d'un axe de rotation (62) s'étendant sensiblement dans la direction longitudinale du tube (12).

12. Endoscope selon l'une des revendications 10 ou 11, **caractérisé en ce que** le raccord d'admission (28), le raccord de décharge (30) et l'optique (60) peuvent tourner par rapport au tube (12) autour de l'axe de rotation (62) s'étendant sensiblement dans la direction longitudinale du tube (12).

13. Endoscope selon l'une des revendications précédentes, **caractérisé en ce qu'**à l'extrémité distale du tube (12) est disposé au moins un outil mobile (16, 17).

14. Endoscope selon la revendication 13, **caractérisé en ce que** l'outil mobile (16, 17) peut tourner par rapport au tube (12) autour d'un axe de rotation s'étendant sensiblement dans la direction longitudinale du tube (12).

15. Endoscope selon l'une des revendications 13 ou 14, **caractérisé en ce que** l'outil comprend au moins une partie de mâchoire (17) mobile par rapport au tube (12).

16. Endoscope selon la revendication 15, **caractérisé en ce qu'**il comprend au moins deux parties de mâchoire (16, 17) mobiles l'une par rapport à l'autre.

17. Endoscope selon l'une des revendications 15 ou 16, **caractérisé en ce que** la au moins une partie de mâchoire mobile (17) peut pivoter par rapport au tube (12) autour d'un axe de pivotement (18) s'étendant sensiblement perpendiculairement à la direction longitudinale du tube (12).

18. Endoscope selon l'une des revendications 15 à 17, **caractérisé en ce qu'**est prévu un dispositif d'actionnement (44, 50) assigné à au moins une partie de mâchoire (17) et que ce dispositif d'actionnement (44, 50) peut tourner par rapport au tube (12).

19. Endoscope selon la revendication 18, **caractérisé en ce que** le dispositif d'actionnement (44, 50) est verrouillable dans une position d'actionnement de la au moins une partie de mâchoire mobile (17).

20. Endoscope selon l'une des revendications 15 à 19, **caractérisé en ce que** sont prévues au moins deux parties de mâchoire (16, 17) qui, dans une position de fermeture dans laquelle les deux parties de mâchoire (16, 17) sont en appui l'une contre l'autre, sont symétriques par rapport à un plan contenant l'axe longitudinal du tube (12).

21. Endoscope selon la revendication 20, **caractérisé en ce que** l'optique (60), dans la zone des au moins deux parties de mâchoire (16, 17), est disposée sur un côté d'une partie de mâchoire fixe (16) et que la partie de mâchoire mobile (17) est disposée sur le côté, opposé à l'optique (60), de la partie de mâchoire fixe (16).

22. Endoscope selon la revendication 21, **caractérisé en ce que** la partie de mâchoire fixe (16) est courbée de façon concave dans la direction de l'optique (60).

23. Endoscope selon l'une des revendications précédentes, **caractérisé en ce que** le tube comprend un segment de tube (12) voisin de l'extrémité distale de l'endoscope (10), susceptible d'être introduit dans un corps humain, que l'axe de symétrie (62) de l'optique dans la zone du segment de tube (12) est parallèle à l'axe de symétrie du tube (12) dans la zone du segment de tube (12) et que les deux axes de symétrie sont distants l'un de l'autre.

24. Endoscope selon l'une des revendications précédentes, **caractérisé en ce qu'**est prévu un dispositif de raccord HF (36) destiné à raccorder l'endoscope (10) à un générateur haute fréquence et que le dispositif de raccord (36) est disposé de manière à pouvoir tourner par rapport au dispositif optique (60).
